# EUROPEAN PATENT APPLICATION

(11) **EP 2 374 888 A1**
(43) Date of publication of application: **12.10.2011**
(21) Application number: 09829420.0
(22) Date of filing: 16.06.2009
(51) Int. Cl.: C12N 15/17, C12N 1/21, C12N 15/70, C12R 1/19

(54) **METHOD FOR PRODUCING HUMAN RECOMBINANT INSULIN**

(30) Priority: 26.11.2008 UA 2008013678
(71) Applicant: Limited Liability Company «Mako», Kiev 01103 (UA)
(72) Inventor: LAZARYEV, Aleksey Pavlovich, Kiev 04208 (UA); LUCIV, Vladimir Romanovich, Kiev 02068 (UA); KOSTETSKII, Igor Evgenievich, Kiev 02166 (UA); LISOVSKYY, Igor Leonidovich, Kievskaya obl. 08132 (UA); LESIK, Igor Pavlovich, Kievskaya obl. 08700 (UA)
(74) Representative: Benatov, Emil Gabriel
(86) International application number: PCT/UA2009/000025
(87) International publication number: WO 2010/062279

(57) **Abstract**

The invention relates to biotechnology and can be used for producing human recombinant insulin for preparing medicinal agents for the treatment of pancreatic diabetes. A variety of recombinant plasmid DNAs which contain an artificial gene and encode the human insulin precursor is proposed. The biosynthesis of a hybrid polypeptide is induced using isopropyl-thiogalactopyranoside so that the post-induction level of the hybrid polypeptide is equal to or greater than 25% of the total cellular protein. According to the claimed procedure, human insulin is produced by cultivating a producer strain containing one of the recombinant plasmids, isolating inclusion bodies, solubilizing and renaturing the fusion protein, and enzymatically degrading and chromatographically purifying said protein. The invention simplifies the process for producing human recombinant insulin and increases the yield thereof.

## Description

The invention is related to biotechnology, gene engineering and medicine and can be utilized in drug production, particularly in manufacturing medicine to treat diabetes mellitus.

Insulin is a protein hormone consisting of an acid A-chain of 21 residues and a basic B-chain of 30 amino acids [1] linked by three disulfides: one intrachain bond (A⁶-A¹¹) and two interchain bonds (A⁷-B⁷ and A²⁰-B¹⁹). While the separate A- and B-chains of insulin can be recombined successfully in vitro [2], a single chain polypeptide (preproinsulin) is normally synthesized in vivo. It contains signal peptide at the B-chain N-termini and C-peptide between A-and B-chains. Following the cleavage of an N-terminal signaling sequence in endoplasmic reticulum the resulting polypeptide folds and is packaged into secretory granules as proinsulin [3]. Processed further by a specific set of proteases proinsulin is then converted to insulin and is secreted into the blood stream to regulate sugar level.

Commercially, human insulin has been produced by transpeptidation wherein an alanine residue at the 30^{th} position of the B-chain of porcine insulin is replaced with a threonine [4]. Since producing of human insulin from porcine insulin is limited by its high cost, recent studies have been focused mostly on processes for producing human insulin by genetic engineering techniques. Three major methods have been utilized for insulin production using microorganisms. Two of them involve *Escherichia coli* with insulin precursor been expressed as a part of large fusion protein in cytoplasm, predominantly in form of inclusion body or been secreted into periplasmic space [5]. The third method utilizes yeasts and insulin precursor is either secreted into the surrounding medium [6] or forms insoluble inclusion body [7]. Process of preparing human insulin by genetic engineering approach comprises the following steps: producing proinsulin in the form of a fusion protein in E. coli, refolding the fusion protein to form correct disulfide bonds; treating the refolded polypeptide with trypsin and carboxypeptidase B; purifying the resultant to obtain insulin. Variation of this approach utilizes also sulphonation of fusion protein and cyanogen bromide cleavage to obtain proinsulin as intermediate product of insulin production [8]. Also, genetic manipulations enable the production of fusion proteins with different amino acid content in proinsulin and/or C-peptide portions of recombinant protein. These manipulations are directed toward increasing the yield of correctly folded proinsulin (preproinsulin). However, the yield of the refolded proinsulin having correct disulfide bonds decreases as the concentration of proinsulin in refolding buffer increases. This is due to the misfolding and some degree of polymerization. Thus, the refolding is crucial step during insulin production. Also, purification of insulin during subsequent steps of insulin production is highly laborious and often costly. Taken all together, the development of new approaches of human insulin production benefits not only the quality of end product, but also saves resources and decreases the product price for consumers. A published method of recombinant human insulin production includes plasmid construction comprising recombinant DNA which code out for proinsulin [9], developing and culturing of *Escherichia coli* strain producing hybrid polypeptide, cell separation and disruption, hybrid polypeptide recovery and enzymatic cleavage followed by isolation of desired product. However, this method utilizes only *Escherichia coli* strain BL21/pIK8-proins, carrying single plasmid DNA pIK8-proins, thus limiting a number of host/expression vector combinations. Furthermore, this method includes treatment of hybrid polypeptide with citraconic anhydride prior to enzymatic cleavage, which adds an additional purification step and decreases yield of desired product. The current invention is aimed on the development of method of recombinant human insulin production, which guarantees increased yield of hybrid polypeptide and manufacturing of desired product with great quality. The main outcome of this method is an improvement of technological effectiveness as result of simplification and elimination of certain manufacturing steps, quantitative increase of end product yield and improved end product quality. All these benefits are owing to the increase of the number of expression vectors, optimization of pre-peptide ammo acid sequence, optimization of refolding process and refinement of cell disruption, inclusion body wash, protein recovery and purification processes. There are close cause-and-effect relations between the array of declared features and technical outcome that is achieved. The present invention relates to the improved process for producing of recombinant human insulin by culturing prokaryotic hosts transformed with DNA sequences encoding those polypeptides. Recombinant hybrid polypeptide comprising a leader sequence attached to proinsulin is synthesized in *Escherichia coli*. Human insulin is produced by the treatment of correctly folded hybrid polypeptides with trypsin and carboxypeptidase B and subsequent purification. According to this invention human recombinant insulin can be manufactured with good reproducibility, while protein recovery and purification steps are significantly improved. The developing of new plasmid DNA constructs allows us to broaden the variety of used strains of microorganisms and to optimize amino acid composition of pre-peptide. The proposed in this method effective technology of hybrid polypeptide concentration by means of adsorption chromatography with fast flow high capacitance resin allows us to improve significantly the productivity of this procedure.

Moreover, the proposed method lacks the blocking of amino groups with citraconic anhydride step prior to enzymatic cleavage. To minimize des-threonine formation trypsinolysis is carried out at high pH values, thus increasing the yield of end product and omitting extra purification steps associated with citraconilation.

In addition, the process of insulin purification has been optimized. It includes ion exchange chromatography and reverse phase high performance liquid chromatography, which allows to produce insulin of 98-99% purity.

So, the problem put by is resolved at the expense of new expression vectors development, improvement of cell disruption and inclusion body wash processes, optimization of refolding and the use of adsorption chromatography for protein concentration, the lack of citraconilation and conduction of trypsinolysis at high pH values to minimize des-threonine formation.

In addition to reported earlier plasmid DNA (pIK8-proins) the current invention represents new plasmids that have some benefits:
- shortened C-peptide (plasmids pMUT12, pISYN2, pCIM61, pDIM07). As result of this modification the yield of end product is increased. Insulin makes 47% of hybrid polypeptide in pIK8-proins expression vector, 68% of hybrid polypeptide when using pMUT12 or pISYN2 plasmids and 82% - in case of using pCIM61 or pDIM07 plasmids.
- new plasmids code out for different hybrid polypeptide, which differ by prepeptide sequence. Amino acid composition of pre-peptides was chosen empirically to change the hybrid polypeptide charge (and isoelectric point, appropriately), thus allowing to use different approaches for protein purification.

DNA encoding hybrid polypeptide (pISYN2 plasmid) has been chemically synthesized taking into account *Escherichia coli* codon frequency usage, this increases the yield of hybrid polypeptide by 40% as compared to similar expression vector comprising human proinsulin cDNA with original codons.

The current invention utilizes very effective technology of high pressure cell disruption (efficiency of cell disruption exceeds 99%), while the efficiency of cell disruption when using well known ultra sonication method is around 40 to 50%. Moreover, this method is cost effective, very productive and yields better quality end product (inclusion body).

Also, preparation of hybrid polypeptide for refolding does not include sulphitolysis step (this step usually decreases the yield of end product and raises production cost). The proposed method of direct refolding of reduced polypeptide is greatly simplified and makes it possible to carry out refolding at protein concentrations of 0.5-1.0 mg/ml, while the reduction of sulphonated polypeptide requires polypeptide concentrations of less then 0.1 mg/ml. In that way the volumes of refolding buffer required for protein renaturation are greatly reduced.

The invention is further illustrated by following figures:

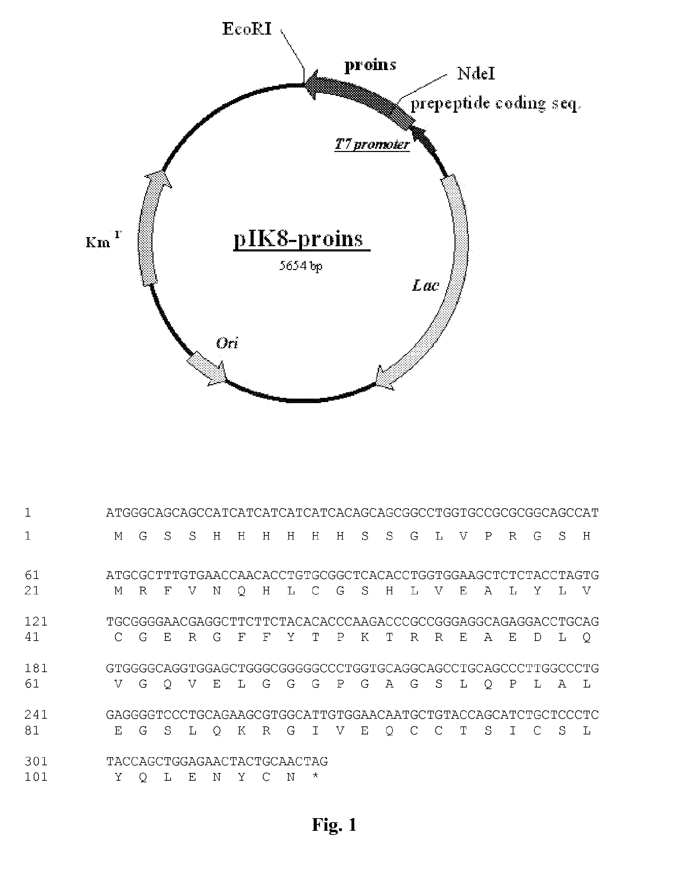

Fig. 1 is a schematic diagram of plasmid DNA pIK8-proins, nucleotide and deduced amino acid sequences of inculin precuror are shown.

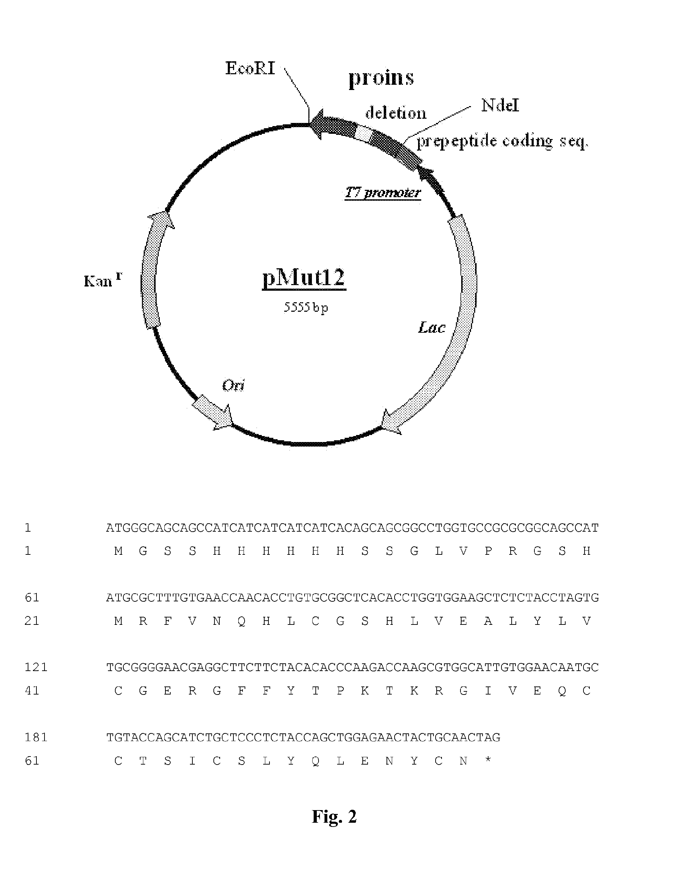

Fig. 2 is a schematic diagram of plasmid DNA pMUT12, nucleotide and deduced amino acid sequences of inculin precuror are shown.

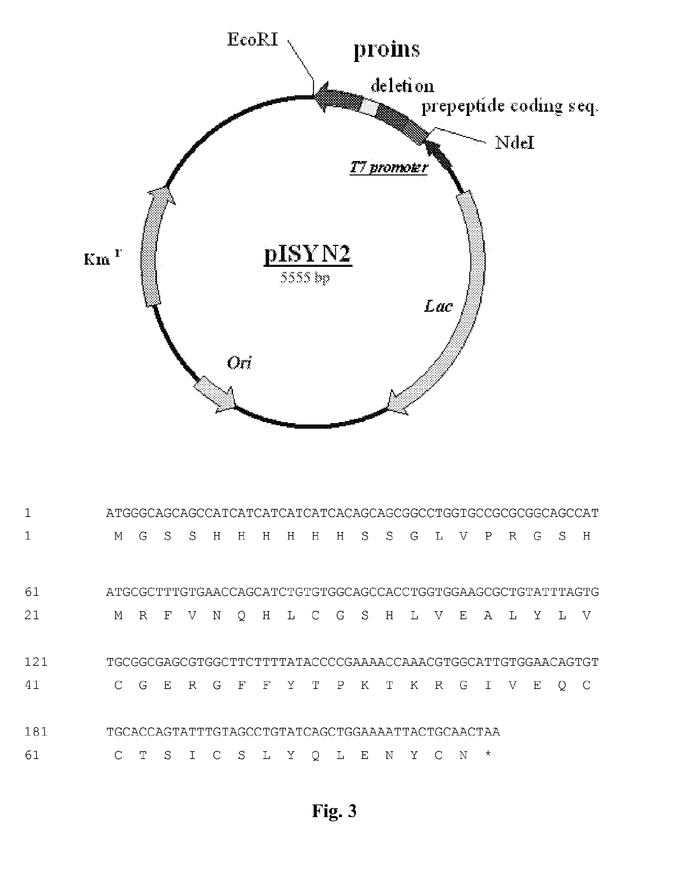

Fig. 3 is a schematic diagram of plasmid DNA pISYN2, nucleotide and deduced amino acid sequences of inculin precuror are shown.

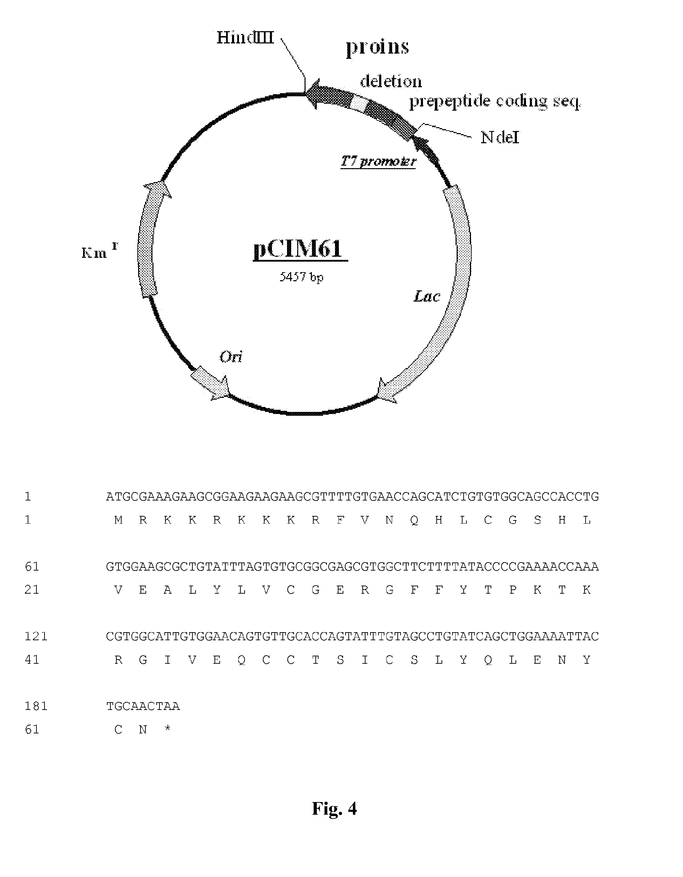

Fig. 4 is a schematic diagram of plasmid DNA pCIM61, nucleotide and deduced amino acid sequences of inculin precuror are shown.

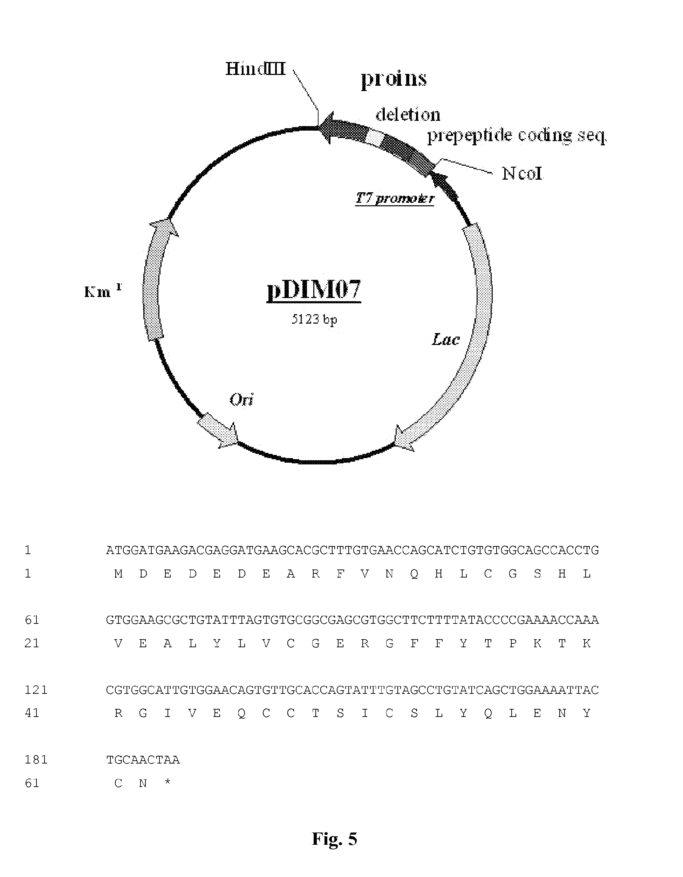

Fig. 5 is a schematic diagram of plasmid DNA pDIM07, nucleotide and deduced amino acid sequences of inculin precuror are shown.

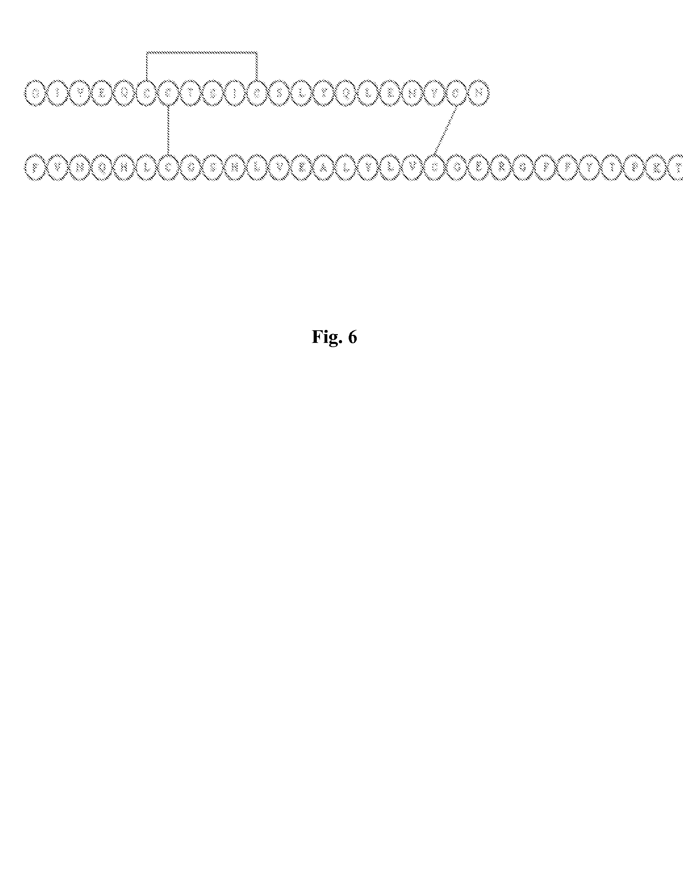

Fig.6 depicts primary structure of human insulin.

The invention is based upon cloning of recombinant DNA encoding human insulin precursor, which is expressed in *Escherichia coli* host cells as inclusion body, followed by recombinant polypeptide recovery and renaturation in conditions that permit correct disulphide bonds formation between cysteine residues. Recombinant polypeptide is further cleaved enzymatically and purified.

Process for producing of hybrid polypeptide comprises the following steps:
a) transforming *Escherichia coli* strains with a recombinant DNA encoding human insulin precursor with trypsin cleavage sites inside of recombinant polypeptide.
b) culturing the transformed host cells in the conditions that permit expression of the recombinant DNA and inclusion body production;
c) cell disruption and recovery of expressed polypeptides;
d) solubilizing the recombinant polypeptide in the presence of denaturant and reducing agent followed by its refolding;
e) enzymatic cleavage of hybrid polypeptide with trypsin and carboxypeptidase B to produce insulin;
f) insulin isolation and purification.

The main objective of current invention is that in the method of recombinant human insulin production by means of construction of recombinant plasmid DNA, which encode proinsulin, the development and culturing of *Escherichia coli* strain producing hybrid polypeptide, cell separation and disruption and polypeptide isolation, its enzymatic conversion followed by purification and isolation of desired product, according to current invention recombinant plasmid DNA fragment which encode hybrid polypeptide comprising amino acid sequence of human proinsulin is a part of expression vector pIK8-proins, or pMUT12, or pISYN2, or pCIM61, or pDIM07 or hybridize to one of the foregoing DNA inserts and which code on expression for human proinsulin precursor, so that recombinant plasmid DNA fragment which encode hybrid polypeptide comprising human proinsulin sequence and which is a part of pIK8-proins plasmid comprises the following sequence: 
1ATGGGCAGCAGCCATCATCATCATCATCACAGCAGCGGCCTGGTGCCGCGCGGCAGCCAT
1 M G S S H H H H H H S S G L V P R G S H

61ATGCGCTTTGTGAACCAACACCTGTGCGGCTCACACCTGGTGGAAGCTCTCTACCTAGTG
21M R F V N Q H L C G S H L V E A L Y L V

121TGCGGGGAACGAGGCTTCTTCTACACACCCAAGACCCGCCGGGAGGCAGAGGACCTGCAG
41C G E R G F F Y T P K T R R E A E D L Q

181GTGGGGCAGGTGGAGCTGGGCGGGGGCCCTGGTGCAGGCAGCCTGCAGCCCTTGGCCCTG
61V G Q V E L G G G P G A G S L Q P L A L

241GAGGGGTCCCTGCAGAAGCGTGGCATTGTGGAACAATGCTGTACCAGCATCTGCTCCCTC
81E G S L Q K R G I V E Q C C T S I C S L

301TACCAGCTGGAGAACTACTGCAACTAG
101Y Q L E N Y C N * ,
 recombinant plasmid DNA fragment which encode hybrid polypeptide comprising human proinsulin sequence and which is a part of pMUT12 plasmid comprises the following sequence: 
1ATGGGCAGCAGCCATCATCATCATCATCACAGCAGCGGCCTGGTGCCGCGCGGCAGCCAT
1 M  G  S  S  H  H  H  H  H  H  S  S  G  L  V  P  R  G  S  H

61ATGCGCTTTGTGAACCAACACCTGTGCGGCTCACACCTGGTGGAAGCTCTCTACCTAGTG
21M  R  F  V  N  Q  H  L  C  G  S  H  L  V  E  A  L  Y  L  V

121TGCGGGGAACGAGGCTTCTTCTACACACCCAAGACCAAGCGTGGCATTGTGGAACAATGC
41C  G  E  R  G  F  F  Y  T  P  K  T  K  R  G  I  V  E  Q  C
 
181TGTACCAGCATCTGCTCCCTCTACCAGCTGGAGAACTACTGCAACTAG
61C T S I C S L Y Q L E N Y C N * ,
 recombinant plasmid DNA fragment which encode hybrid polypeptide comprising human proinsulin sequence and which is a part of pISYN2 plasmid comprises the following sequence: 
1ATGGGCAGCAGCCATCATCATCATCATCACAGCAGCGGCCTGGTGCCGCGCGGCAGCCAT
1 M G S S H H H H H H S S G L V P R G S H

61ATGCGCTTTGTGAACCAGCATCTGTGTGGCAGCCACCTGGTGGAAGCGCTGTATTTAGTG
21M R F V N Q H L C G S H L V E A L Y L V

121TGCGGCGAGCGTGGCTTCTTTTATACCCCGAAAACCAAACGTGGCATTGTGGAACAGTGT
41C G E R G F F Y T P K T K R G I V E Q C

181TGCACCAGTATTTGTAGCCTGTATCAGCTGGAAAATTACTGCAACTAA
61C T S I C S L Y Q L E N Y C N * ,
 recombinant plasmid DNA fragment which encode hybrid polypeptide comprising human proinsulin sequence and which is a part of pCIM61 plasmid comprises the following sequence: 
1ATGCGAAAGAAGCGGAAGAAGAAGCGTTTTGTGAACCAGCATCTGTGTGGCAGCCACCTG
1 M R K K R K K K R F V N Q H L C G S H L

61GTGGAAGCGCTGTATTTAGTGTGCGGCGAGCGTGGCTTCTTTTATACCCCGAAAACCAAA
21V E A L Y L V C G E R G F F Y T P K T K

121CGTGGCATTGTGGAACAGTGTTGCACCAGTATTTGTAGCCTGTATCAGCTGGAAAATTAC
41R G I V E Q C C T S I C S L Y Q L E N Y

181TGCAACTAA
61C N * ,
 recombinant plasmid DNA fragment which encode hybrid polypeptide comprising human proinsulin sequence and which is a part of pDIM07 plasmid comprises the following sequence:

1ATGGATGAAGACGAGGATGAAGCACGCTTTGTGAACCAGCATCTGTGTGGCAGCCACCTG
1 M  D  E  D  E  D  E  A  R  F  V  N  Q  H  L  C  G  S  H  L

61GTGGAAGCGCTGTATTTAGTGTGCGGCGAGCGTGGCTTCTTTTATACCCCGAAAACCAAA
21V  E  A  L  Y  L  V  C  G  E  R  G  F  F  Y  T  P  K  T  K

121CGTGGCATTGTGGAACAGTGTTGCACCAGTATTTGTAGCCTGTATCAGCTGGAAAATTAC
41R  G  I  V  E  Q  C  C  T  S  I  C  S  L  Y  Q  L  E  N  Y

181TGCAACTAA
61C  N  * .

*Escherichia coli* strain development utilizes expression vectors comprising nucleotide sequence encoding one or several recombinant polypeptides, here the transcription of said nucleotide sequence encoding one or several recombinant polypeptides is placed under the control of an inducible expression system, then the culturing of transformed *Escherichia coli* strain is carried out followed by the induction of expression to permit the synthesis of recombinant polypeptide or polypeptides in host cells and the recovery of produced recombinant polypeptides.

Inclusion body recovery is carried out by disrupting the cell wall of the bacterial cell or fragments thereof, separating intracellular precipitate from the lysate and washing the intracellular precipitate with nonionic detergent, its further conversion include solubilizing the precipitate in a buffer solution containing the denaturant, treatment of the hybrid polypeptide with reducing agent, refolding the hybrid polypeptide and concentrating the hybrid polypeptide by means of adsorption chromatography or ultrafiltration.

Proteolytic conversion of insulin precursor is performed either by simulteneous treatment with trypsin and carboxypeptidase B or by separate treatment with these two enzymes purifying the resultant using preferably ion exchange chromatography and/or high performance liquid chromatography.

The method is carried out as described further.

A wide variety of host/expression vector combinations may be utilized in this invention that permit the optimal production of the human insulin. For example, useful expression vectors may consist of promoter, a translation start signal, human proinsulin cDNA with or without modifications introduced into C-peptide sequence and optional prepeptide separated from proinsulin by trypsin cleavage site. A preferred recombinant DNA molecule of the present invention is a plasmid which contains a DNA insert as described above. Preferred plasmids of the present invention include pIK8-proins, pMUT12, pISYN2 and derivatives thereof.

Also embraced within the present invention is a process of the manufacturing of recombinant DNA molecule. This process includes providing a DNA insert which corresponds to all, part, analogues, homologues or precursors of a polypeptide which is insulin and introducing into a cloning vehicle this DNA insert.

Preferably said DNA sequence is introduced into the cloning vehicle in correct reading frame with an expression control sequence.

In a further embodiment of the present invention host cells are transformed with at least one recombinant DNA molecule capable of expressing all, part or parts, or precursors of human insulin or a polypeptide having similar immunological or biological activity to insulin.

Useful expression hosts include well known prokaryotic hosts, such as strains of *Escherichia coli*, including but not limited to the following strains: *Escherichia coli* HB101, *Escherichia coli* X1776, *Escherichia coli* X2282, *Escherichia coli* DH5α, *Escherichia coli* JM103, *Escherichia coli* BL21.

The bacterial host cells are typically cultured in a liquid growth medium for production of insulin precursor polypeptide under conditions appropriate to the host cells and expression vector. Preferably, the host cells are cultured in a bacterial fermenter to maximize production, but any convenient method of culture is acceptable (e.g., shaken flask, especially for cultures of less than a liter in volume). The exact growing conditions, timing and rate of media supplementation, culture medium pH, temperature and dissolved oxygen level and addition of inducing agent (where appropriate) vary according to the identity of the host cells and the expression construct.

After the bacterial host cells are cultured to the desired density and any necessary induction of expression is completed, the cells are collected. Collection is typically done by centrifugation of the growth medium, although any other convenient technique like ultrafiltration may be used. At this point, the collected bacterial host cells may be immediately processed in accordance with the invention, or it may be frozen for processing at a later time.

The cells of the cell paste are then lysed to release insulin precursor polypeptide-containing inclusion bodies. The cells are suspended in a buffer at about pH 6.0 to 9.0, using an ionic strength of the order of about 0.01 M to 2 M. Any suitable salt, including NaCl can be used to maintain an appropriate ionic strength level. Preferably, the cells are lysed under conditions in which the cellular debris is sufficiently disrupted that it fails to appear in the pellet under low speed centrifugation. The cells are lysed by common techniques such as mechanical methods (such as freeze/thaw cycling, the use of a Microfludizer, a French press, or a sonic oscillator), or by enzymatic methods (such as treatment with lysozyme). It is generally recommended to perform cell lysis under conditions of reduced temperature (i.e., less than about 20.degree. C.)

Inclusion bodies are collected from the lysed cell paste using any convenient techniques (such as centrifugation), then washed if needed. Inclusion bodies are typically washed by resuspending the inclusion bodies in a buffer with a detergent added (such as Triton X-100), then recollecting the inclusion bodies. The washed inclusion bodies are then dissolved in solubilization buffer, which comprises high concentration of a chaotrophe (such as urea or guanidine hydrochloride), reduced agent and optional pH.

The concentration of urea in the solubilization buffer is between 6M and 8M, preferably 7M and guanidine hydrochloride concentration is 5 to 7M, preferably 6M.

The pH of the solubilization buffer ranges from 7.5 to 11.0, preferably 8.5 to 9.0. Any pH buffering agent can be used (such as Tris, HEPES, MOPS, tricine and the like). The pH buffering agent is added to solubilization buffer at the concentration that provides effective pH buffering, such as from 10 to about 100mM, preferably 20mM.

Reducing reagents are included in the solubilization buffer to reduce disulfide bonds and to maintain cysteine residues in their reduced form. Useful reducing reagents include beta-mercaptoethanol, dithiothreitol, and the like. Optional concentration of beta-mercaptoethanol is between 20 and 200mM, preferably between 100 and 150mM.

The solubilization buffer optionally contains additional components such as cation chelator like EDTA. EDTA is added to the solubilization buffer at a concentration of about 0.5 to about 5mM, preferably at 1mM to about 2mM. Additionally, a glycine (or other amino acids) may be added to reduce or eliminate free-radical-mediated protein damage. Glycine concentration ranges from 5 to 100mM, preferably from 10 to 20mM.

The solubilization of inclusion body is generally done over period from about six hours to about 24 hours, and preferably about 8 hours. The solubilization may be carried out at ambient or reduced temperature, commonly at about 4 degree to about 24 degree C, preferably at about 20 degree C. After the solubilization of inclusion body is complete, the reduced polypeptide solution is clarified to remove insoluble debris. Clarification can be performed by using high speed centrifugation.

The reduced proinsulin precursor is then rapidly diluted with refolding buffer. The dilution is performed by adding inclusion body solution into the refolding buffer or by simultaneous delivery into the refolding vessel of refolding buffer and inclusion body solution. The inclusion body solution may be diluted about 10 to about 200 fold, most preferably 100 fold with refolding buffer. The final protein concentration after dilution may be about 0.01 mg/ml to about 5 mg/ml, preferably about 1 mg/ml.

The refolding buffer generally contains a pH buffer, a disulfide reshuffling redox system, a divalent cation chelator, free-radical scavengers and some other low molecular weight additives. The refolding process is carried out at the temperature from about 4 degree C to about 24 degree C, preferably at about 10 degree C. The incubation time is generally from about 2 hours to about 20 hours, preferably about 6 hours. Main components of refolding buffer include glycine at concentration from about 1mM to 100mM, preferably 10mM; cystine at concentration from about 0.5mM to 20mM; EDTA from about 0.1mM to 5mM; glycerol from about 1% to 20%.

Following the refolding reaction, properly refolded insulin precursor may be concentrated, further purified, and the protein may be proteolytically processed (e.g., with trypsin and carboxypeptidase B) to produce mature insulin. Concentration of the refolded protein may be accomplished using any convenient technique, such as ultrafiltration or chromatography (e.g., ionexchange, hydrophobic interaction, or affinity chromatography) and the like. The concentration step may also include a buffer exchange process, if so desired. It is preferred that concentration to be carried out at reduced temperature (e.g., about 4-10 degree C).

Proteolytic conversion of insulin precursor is performed either by simultaneous treatment with trypsin and carboxypeptidase B or by separate treatment with these two enzymes. Trypsin is used at 1:200 to about 1:20,000 (enzyme:substrate, weight:weight) ratio, while carboxypeptidase B is used at 1:100 to about 1:5,000 ratio. The proteolytic conversion is carried out in buffer with pH 7.5 to about 11.0, preferably at pH 10.5; the addition of Ca²⁺, Zn²⁺, Mn²⁺, Mg²⁺ may benefit the reaction. Incubation temperature ranges from 0°C to about 30°C, preferably is around 6°C, incubation time is between 1 and 24 hours, preferably around 14 hours.

After proteolytic conversion, the biologically active insulin is purified.

The following examples illustrate the invention. It should be understood that these examples are for illustrative purposes only and should not be considered as limiting this invention in any way.

### Example 1.

### Construction of plasmid pIK8-proins.

A human insulin cDNA was amplified from human pancreas single-stranded cDNA using the polymerase chain reaction (PCR) technique and primers P1 and P2:
primer P1 (forward primer)
5'-GCCGATATGCGATTTGTGAACCAACACCTGTG-3'
primer P2 (reverse primer)
5'-TGGAATTCCTAGTTGCAGTAGTTCTCCAGC-3'

Primer P1 was designed to encode a fragment of human insulin B-chain starting with Arg²² of human preproinsulin and Ndel site (boxed) for cloning purposes. The reverse primer (P2) complements amino terminus of insulin A-chain and EcoRI site (boxed) for cloning purposes. The PCR reactions contained 25 pmoles of each oligo primer, 1×PCR buffer, 200 µM concentration of each of the four nucleotides (dA, dC, dG and dT), 2ng of single-stranded cDNA, 5.0 units of Taq polymerase. The PCR reaction conditions were: 95°C for 3 minutes, 35 cycles of (95°C for 1 minute; 65°C for 30 seconds; 72°C for 30 seconds), followed by 5 minutes at 72°C. The approximate 280bp PCR product was gel-purified and cloned into pTA cloning vector. The ligation mixture was transformed into *Escherichia coli* strain DH5alpha and transformants selected on LB plates containing ampicillin. Several colonies were grown overnight in LB media and plasmid DNA isolated using Wizard minipreps DNA isolation kit. Clone IK8 was determined to have the correct DNA sequence.

For expression in *Escherichia coli*, plasmid DNA isolated from clone IK8 was digested with NdeI and EcoRI, the approximate 270bp fragment was gel purified, and cloned into plasmid pET28a that had been digested with the same enzymes and treated with calf intestinal alkaline phosphatase. The ligation mixture was transformed into *Escherichia coli* DH5alpha and transformants selected on LB kanamycin plates. Plasmid DNA was isolated from several transformants and screened by digestion with NdeI and EcoRI. A correct clone was identified and named pIK8-proins. This plasmid was further transformed into *Escherichia coli* strains like JM109 or BL21.

### Example 2.

### Construction of plasmid pMUT12.

Plasmid DNA pIK8-proins was used as a template in site directed mutagenesis using PCR and the following primers:
M1: 5' - CTTCTACACACCCAAGACCAAGCGTGGCATTGTGGA ACAATGCTG -3'
M2: 5' -CAGCATTGTTCCACAATGCCACGCTTGGTCTTGGGTGT GTAGAAG -3'

Following denaturation at 96°C for 3 min 30 cycles of PCR were performed using 5U rTth DNA polymerase. PCR conditions were as following: 94°C, 30s; 59°C, 30s; 72°C, 6 min and the final extension at 72°C for 10 min. PCR product was purified with Zymoclean PCR purification kit and digested with 10 U of Dpnl - to remove an original template. After another round of column purification a 2mkl aliquot was transformed into *Escherichia coli* strain DH5alpha and transformants selected on LB plates containing kanamycin. Several colonies were grown overnight in LB media and plasmid DNA isolated using Wizard minipreps DNA isolation kit. Clone MUT12 was determined to have the correct DNA sequence. For expression in *Escherichia coli* this plasmid was further transformed into *Escherichia coli* strains like JM109 or BL21.

### Example 3

### Construction of plasmid pISYN2

To construct pCSYN61 we first synthesized 5 oligodeoxynucleotides using the reported amino acid sequence of human insulin [10]. In these syntheses, we considered the codon usage in highly expressed genes of *Escherichia coli* [11] and *Escherichia coli* tRNA abundance [12]. We also included endonuclease recognition sites at various positions along our oligonucleotide sequences for cloning purpose.

Primer sequences:
S1: 5'- CATATGCGCTTTGTGAACCAG-3'
S2: 5'-AAGCCACGCTCGCCGCACACTAAATACAGCGCTTCCA CCAGGTGGCTGCCACACAGATGCTGGTTCACAAAGCGCATATG-3'
S3: 5'-CGGCGAGCGTGGCTTCTTTTATACCCCGAAAACCAAA CGTGGCATTGTGGAACAGTGTTGCACCAGTATTTGTAGCCTGT-3'
S4: 5'-CAGGCGTGAATTCTTAGTTGCAGTAATTTTCCAGCTG ATACAGGCTACAAA TACTGGTGC-3'
S5: 5'- CAGGCGTGAATTCTTAGTTGC-3'

Mixture of primers S1, S2, S3, S4 (final concentration 2mkm each) in PCR buffer with 5U of rTth polymerase were heated to 94°C for 1 min, cooled to 62°C and the reaction was carried out at 72°C for 2 min to fill the gaps. Next 20 cycles of amplification was performed in following conditions: 94°C, 15s; 62°C, 30s; 72°C, 30s. Two mkl of the above reaction mixture was used as a template to amplify proinsulin cDNA in the presence of S1 and S5 primers. PCR fragment of approximately 180bp was gel purified and cloned in to pTA-cloning vector. Clone IS2 was determined to have the correct DNA sequence. For expression in *Escherichia coli*, plasmid DNA isolated from clone IS2 was digested with NdeI and EcoRI, the approximate 170bp fragment was gel purified, and cloned into plasmid pET28a that had been digested with the same enzymes and treated with calf intestinal alkaline phosphatase. The ligation mixture was transformed into *Escherichia coli* DH5alpha and transformants selected on LB kanamycin plates. Plasmid DNA was isolated from several transformants and screened by digestion with NdeI and EcoRI. A correct clone was identified and named pISYN2. This plasmid was transformed into *Escherichia coli* strains like JM109 or BL21.

### Example 4

### Construction of plasmid pCIM61

Plasmid DNA pMUT12 was used as a template to amplify human proinsulin cDNAmini with modified pre-peptide. PCR reaction was carried out in the conditions described for example 1 with the following primers:
Primer C (forward):
5'-GCCATATGCGAAAGAAGCGGAAGAAGAAGCGTTTTGTG AACACCTGTG-3'
Primer IL4 (Reverse):
5'-CCGCAAGCTTTTAGTTGCAGTAGTTCTCCAGCTGG-3'

The approximate 210bp PCR product was gel-purified and cloned into pTA cloning vector. The ligation mixture was transformed into *Escherichia coli* strain DH5alpha and transformants selected on LB plates containing ampicillin. Clone CIM6 was determined to have the correct DNA sequence.

For expression in *Escherichia coli*, plasmid DNA isolated from clone CIM6 was digested with NdeI and HindIII, the approximate 200bp fragment was gel purified, and cloned into plasmid pET39a that had been digested with the same enzymes. The ligation mixture was transformed into *Escherichia coli* DH5alpha and transformants selected on LB kanamycin plates. Plasmid DNA was isolated from several transformants and screened by digestion with NdeI and HindIII. A correct clone was identified and named pCIM61. This plasmid was transformed into *Escherichia coli* strains like JM109 or BL21.

### Example 5

### Construction of plasmid pDIM07

Plasmid DNA pMUT12 was used as a template to amplify human proinsulin cDNAmini with modified prepeptide. PCR reaction was carried out in the conditions described for example1 with the following primers:
Primer D (forward):
5'- TACCATGGATGAAGACGAGGATGAAGCACGCTTTGTGAA CCAACACCTGTG-3'
Primer IL4(Reverse):
5'-CCGCAAGCTTTTAGTTGCAGTAGTTCTCCAGCTGG-3'

The approximate 210bp PCR product was gel-purified and cloned into pTA cloning vector. The ligation mixture was transformed into *Escherichia coli* strain DH5alpha and transformants selected on LB plates containing ampicillin.. Clone DIM1 was determined to have the correct DNA sequence.

For expression in E. coli, plasmid DNA isolated from clone DIM1 was digested with NcoI and HindIII, the approximate 200bp fragment was gel purified, and cloned into plasmid pET28a that had been digested with the same enzymes. The ligation mixture was transformed into *Escherichia coli* DH5alpha and transformants selected on LB kanamycin plates. Plasmid DNA was isolated from several transformants and screened by digestion with NcoI and HindIII. A correct clone was identified and named pDIM07. This plasmid was transformed into *Escherichia coli* strains like JM109 or BL21.

### Example 6

### Expression of insulin precursor proteins

The plasmids confirmed to comprise the DNA fragment coding for insulin precursor (pIK8-proins, pMUT12, pCIM61, pISYN2, pDIM07 etc) were used to transform the expression host cell E. coli BL21(DE3) in accordance with the same procedures as above, and the kanamycin-resistant colonies were selected. E. coli BL21(DE3) cells transformed with plasmids pIK8-proins, pISYN2 and pCIM61 were deposited at the Ukrainian Center of Microorganisms with the accession numbers of IMB B-7169, IMB B-7230 and IMB B-7251 appropriately and at the Czech Collection of Microorganisms with the accession numbers of CCM 7511, CCM 7568 and CCM 7567 appropriately under the terms of the Budapest Treaty on the International Recognition of the Deposit of Microorganism for the Purpose of Patent Procedure.

The colonies selected above were inoculated in 1 ml of LB medium (10g bacto tryptone, 5g bacto yeast extract and 10g NaCl per L) and then cultured at 37°C for more than 12 hours. The culture was transferred to 50ml of LB medium containing 30µg/ml of kanamycin and, when the OD₆₀₀ of the culture was 0.5 to 0.8, IPTG (isopropyl-β-D-thiogalactopyranoside) was added to the culture to a final concentration of 1mM. The culture was continued at 37°C for 4 hours with shaking at 200 rpm, and centrifuged at 4,000 rpm for 15 min to obtain *Escherichia coli* cell pellets. The cells were lysed by heating for 5min at 95°C in protein loading buffer and lysate was analyzed by SDS gel electrophoresis according to standard procedure [13].

### Example 7

### Fermentation and growth conditions

### 1. Working bank

Single colony of *Escherichia coli* strain BL21 harboring either of above mentioned plasmids was grown in LB medium (10g/L bacto tryptone, 5/L yeast extract and 5g/L NaCl) or its analog supplemented with appropriate antibiotic. The cultures were then diluted with freezing medium and stored at - 80° C or at -170°C.

### 2. Inoculum

Sterile LB medium supplemented with 30 µg/ml of kanamycin or 50 µg/ml of ampicillin in a shake flask was inoculated from working bank and incubated 15 hours on a shaker at 37° C and approximately 250 rpm. If needed, subsequent stages in inoculum propagation were carried out in stirred aerated fermenters. Sterile medium was inoculated with 5-10% flask culture, and incubated 5-8 hours at 37° C, pH 6.9±0.5 with agitation and aeration to maintain the dissolved oxygen level above 20% air saturation.

### 3. Production

The production medium was inoculated with 1-10% inoculum culture and incubated at 37° C. Agitation-aeration rates were set to maintain the dissolved oxygen level above 20% air saturation. The pH was maintained at 6.9±0.5 with NH₄OH Propinol B400 was used as antifoaming agent.

### Production medium:

| Bacto tryptone | 3.5 g/L |
|---|---|
| Yeast extract | 3.5 g/L |
| glucose | 2.0 g/L |
| NaCl | 1.0 g/L |
| (NH₄)Cl | 3.0 g/L |
| K₂HPO₄ | 4.0 g/L |
| (NH₄)H₂PO₄ | 2.0 g/L |
| (NH₄)₂SO₄ | 2.0 g/L |
| K₂SO₄ | 3.0 g/L |
| MgSO₄ | 1.0 g/L |
| thiamine | 5 mg/L |
| Trace elements | 2ml/L |
| kanamycin | 30 mg/L |

### Trace elements solution:

| | |
|---|---|
| FeSO₄*7H₂O | 10 g/L |
| ZnSO₄*7H₂O | 2,25 g/L |
| CuSO₄*7H₂O | 1,0 g/L |
| MnCl₂ | 0,25 g/L |
| Na₂B₄O₇*10H₂O | 0,25 g/L |
| CaCl₂*2H₂O | 2,0 g/L |
| (NH₄)₆Mo₇O₂₄ | 0,1 g/L |
| 0.75 M EDTA | 100m1/L |
| Citric acid | 60 g/L |

Sterile solution of 50% glucose was infused to supply carbon source. Once cell concentration reached an OD₆₀₀ of about 30, sterile solution of IPTG (final concentration 1mM) was infused and growth continued for 6 hours, cell concentration reached an approximate OD₆₆₀ of 50. The culture was then chilled and cells were recovered by centrifugation.

### Example 8

### Purification of inclusion bodies

The bacterial wet cake was resuspended (1:10 ratio) in cold buffer containing 20mM Tris, pH 7.5, 1mM EDTA and 100mM NaCl. The cell suspension was passed through cell disruptor at 17,000 psi and inclusion bodies recovered by centrifugation.

The pellet containing inclusion bodies and bacterial fragments was washed with 20-fold volume of cold (+10°C) washing buffer containing 1.0 % Triton X100, 20mM Tris, pH 8.0, 2mM EDTA, 100mM NaCl. The pellet was recovered by centrifugation at 14,000 rpm. This washing and recovery steps were repeated twice.

1.5 kg of wet inclusion bodies containing approximately 1.0 kg of hybrid polypeptide as determined by Bradford method were dissolved in 15 L of 8M urea containing 50mM glycine, pH 8.0, 2mM EDTA, 150mM (β-mercaptoethanol, incubation continued for 6hr at room temperature. The solution was clarified by high speed centrifugation and reduced insulin precursor was used for refolding.

### Example 9

### Purification of inclusion bodies

The inclusion bodies water suspension buffered with 20 mM Tris-HCl, pH7.5 and 10 mM MgSO₄ with total volum of 60 L containing 12 kg of protein was treated with lysozyme (0.1 mg/ml), RNase (0.5 U/ml) and DNase (0.5 U/ml) for 3 hours at 25°C.

After enzymatic treatment of inclusion bodies total nucleic acid content was decreased from 656 mg/ml to 198 mg/ml.

Inclusion bodies were washed from enzymes excess using high speed centrifugation and dissolved in 100 L of buffered solution containing 8M urea, 20 mM glycine, pH 8.5; 1 mM EDTA and 150 mM (β-mercaptoethanol. Protein reduction was carried out for 4 hours at room temperature. Then the solution was clarified using high speed centrifugation and the reduced insulin precursor was refolded.

### Example 10

### Refolding of hybrid polypeptide

The reservoir is filled with refolding buffer (50mM glycine, 2mM EDTA, 5mM cystine, 10% glycerol, pH 11.2) and chilled to 5°C. Then the clarified protein solution and the refolding buffer are mixed rapidly in a ratio 1:70 (v/v) by connecting two chambers to a mixing cell, refolding reaction mixture is transferred into refolding vessel for 10 hrs at 5°C with slow stirring. The protein concentration in refolding vessel is 1.0 g/L as determined by Bradford method.

### Concentration

Concentration of refolded protein is routinely carried out by ultrafiltration or using adsorption chromatography. A 450x500 mm chromatography column was packed with 30 L of polymeric resin Amberchrom CG-300M. Refolding reaction mixture was loaded on the column at a flow rate 4 L/min and the column was washed with 3 column volumes of the equilibrium buffer. The protein was eluted with 40% 2-propanol. Then, the eluent was analyzed by HPLC, which revealed that purity was 45% or more and recovery rate was 96%.

### Example 11

### Refolding of hybrid polypeptide

Refolding vessels were filled with cold (10°C) buffer containing 10 mM glycine, 1 mM EDTA, 2 mM cystine, 1 g/L PEG 1500, pH 11.2 and 50 L of reduced protein was mixed with 5000 L of refolding buffer. Protein concentration in the refolding mixture was estimated as 0.5 g/L.

After 6 hours Zn²⁺ was added to the refolding mixture to final concentration of 3 mM and pH was adjusted to 6.2. Protein precipitate was recovered using disc bowl separator and dissolved in 6 M urea.

### Example 12

### Enzymatic conversion

Insulin precursor in 50mM Tris buffer, pH 8.0 was converted to human insulin by incubation with trypsin (1:8,000; w/w) and carboxypeptidase B (1:2,000; w/w) in presence of 1.5M of Zn²⁺. Reaction was carried out 16 hrs at 8°C and stopped by adjusting pH to 2.5 with 6M HCl. HPLC analysis revealed that purity was 69% or more. Insulin can be precipitated from reaction mixture with ammonium sulphate or sodium chloride (final concentration 20%).

### Example 13

### Ion exchange chromatography

300 g of insulin precipitate was dissolved in 19 L of buffer containing 50mM Tris, 1mM EDTA, pH 8.3; 32% 2-propanol. The insulin solution was filtered through 0.5 µm filter and loaded on ion exchange column at flow rate 320 ml/min. The column was packed with 19 L of resin Matrex PEI-300. After insulin adsorption the column was washed with 2 column volumes of the equilibrium buffer. The protein was eluted by a concentration gradient by using the equilibrium buffer containing 0-0.3M NaCl. Then, the eluents collected at 0.1-0.2M NaCl were analyzed by HPLC, which revealed that purity was 85% or more and recovery rate was 91%.

### Example 14

### HPLC insulin purification

Insulin precipitate after ion exchange chromatography was dissolved in buffer solution containing 0.05 M of sodium acetate, pH 3.0 and 10% of 2-propanol and filtered through 0.2 µm filter. Insulin solution was loaded on 200x600 mm column packed with 19 L of Diasogel SP-120-20-ODS-AP matrix equilibrated with 40 L of loading buffer. The protein was eluted by a concentration gradient (10-28%) of 2-propanol in the loading buffer. Matrix regeneration was done with the solution containing 50% of 2-propanol. Analytical HPLC revealed that insulin purity was 98% or greater.

### REFERENCES

1. Brandenburg D, Wollmer A. Insulin: chemistry, structure, and function of insulin and related hormones : proceedings of the Second International Insulin Symposium, Aachen, Germany, September 4-7, 1979. Publisher: New York : W. de Gruyter, 1980.
2. Fischer M, Fytlovitch S, Amit B, Wortzel A, Beck Y. A constitutive expression vector system driven by the deo P1P2 promoters of Escherichia coli. Appl Microbiol Biotechnol. 1990, 33: 424-8.
3. Davidson HW, Rhodes CJ, Hutton JC. Intraorganellar calcium and pH control proinsulin cleavage in the pancreatic beta cell via two distinct site-specific endopeptidases. Nature,1988,333:93-96.
4. Markussen J. Human insulin by tryptic transpeptidations of porcine insulin and biosynthetic precursors. Boston : MTP Press, 1987.
5. Chance RE, Kroeff EP, Hoffmann JA, Frank BH. Chemical, physical, and biologic properties of biosynthetic human insulin. Diabetes Care, 1981,2:147-54.
6. Thim L, Hansen MT, Norris K, Hoegh I, Boel E, Forstrom J, Ammerer G, and Fiil NP. Secretion and Processing of Insulin Precursors in Yeast. PNAS, 1986, 83: 6766-6770.
7. Cousens LS, Shuster JR, Gallegos C, Ku LL, Stempien MM, Urdea MS, Sanchez-Pescador R, Taylor A, Tekamp-Olson P. High level expression of proinsulin in the yeast, Saccharomyces cerevisiae. Gene, 1987, 61:265-275.
8. Cowley DJ, Mackin RB. Expression, purification and characterization of recombinant human proinsulin. FEBS Letters, 1997, 402: 124-130.
9. Lazarev AP, Lesik IP, Kostetskii IE, Lisovskiy IL, Rybachuk VM, Stadnik VI. A method for producing recombinant human insulin.Patent of Ukraine for invention Nº 76661. C12N 15/17, C12N 1/21, publ. 2006.
10. Le Lay J, Matsuoka TA, Henderson and Stein R. Identification of a novel PDX-1 binding site in the human insulin gene enhancer J. Biol. Chem., 2004, 279: 22228-22235.
11. Grantham R, Gautier C, Gouy M. Codon frequencies in 119 individual genes confirm consistent choices of degenerate bases according to genome type. Nucleic Acids Res.,1980, 8:1893-1912.
12. Ikemura T T. Correlation between the abundance of Escherichia coli transfer RNAs and the occurrence of the respective codons in its protein genes: a proposal for a synonymous codon choice that is optimal for the E. coli translational system. J. Mol. Biol.,1981, 151:389-409.
13. Sambrook J, Fritsch E F and Maniatis T. Molecular Cloning: A Laboratory Manual, 2nd edition, Cold Spring Harbor Laboratory Press, 1989.

## Claims

1. A method of recombinant human insulin production by means of construction of recombinant plasmid DNA, which encode proinsulin, the development and culturing of *Escherichia coli* strain producing hybrid polypeptide, cell separation and disruption and polypeptide isolation, its enzymatic conversion followed by purification and isolation of desired product, a method **characterized in that** recombinant plasmid DNA fragment which encode hybrid polypeptide comprising amino acid sequence of human proinsulin is a part of expression vector pIK8-proins, or pMUT12, or pISYN2, or pCIM61, or pDIM07 or hybridize to one of the foregoing DNA inserts and which code on expression for human proinsulin precursor, so that recombinant plasmid DNA fragment which encode hybrid polypeptide comprising human proinsulin sequence and which is a part of pIK8-proins plasmid has the following sequence: 
1          ATGGGCAGCAGCCATCATCATCATCATCACAGCAGCGGCCTGGTGCCGCGCGGCAGCCAT
1           M   G   S   S   H   H   H   H   H   H   S   S   G   L   V   P   R   G   S   H

61          ATGCGCTTTGTGAACCAACACCTGTGCGGCTCACACCTGGTGGAAGCTCTCTACCTAGTG
21          M   R   F   V   N   Q   H   L   C   G   S   H   L   V   E   A   L   Y   L   V

121         TGCGGGGAACGAGGCTTCTTCTACACACCCAAGACCCGCCGGGAGGCAGAGGACCTGCAG
41          C   G   E   R   G   F   F   Y   T   P   K   T   R   R   E   A   E   D   L   Q

181         GTGGGGCAGGTGGAGCTGGGCGGGGGCCCTGGTGCAGGCAGCCTGCAGCCCTTGGCCCTG
61          V   G   Q   V   E   L   G   G   G   P   G   A   G   S   L   Q   P   L   A   L

241         GAGGGGTCCCTGCAGAAGCGTGGCATTGTGGAACAATGCTGTACCAGCATCTGCTCCCTC
81          E   G   S   L   Q   K   R   G   I   V   E   Q   C   C   T   S   I   C   S   L

301         TACCAGCTGGAGAACTACTGCAACTAG
101         Y   Q   L   E   N   Y   C   N   *,
 recombinant plasmid DNA fragment which encode hybrid polypeptide comprising human proinsulin sequence and which is a part of pMUT12 plasmid has the following sequence: 
1          ATGGGCAGCAGCCATCATCATCATCATCACAGCAGCGGCCTGGTGCCGCGCGGCAGCCAT
1           M  G  S  S  H  H  H  H  H  H  S  S  G  L  V  P  R  G  S  H

61          ATGCGCTTTGTGAACCAACACCTGTGCGGCTCACACCTGGTGGAAGCTCTCTACCTAGTG
21          M  R  F  V  N  Q  H  L  C  G  S  H  L  V  E  A  L  Y  L  V

121         TGCGGGGAACGAGGCTTCTTCTACACACCCAAGACCAAGCGTGGCATTGTGGAACAATGC
41          C  G  E  R  G  F  F  Y  T  P  K  T  K  R  G  I  V  E  Q  C

181         TGTACCAGCATCTGCTCCCTCTACCAGCTGGAGAACTACTGCAACTAG
61          C  T  S  I  C  S  L  Y  Q  L  E  N  Y  C  N  *  ,
 recombinant plasmid DNA fragment which encode hybrid polypeptide comprising human proinsulin sequence and which is a part of pISYN2 plasmid has the following sequence: 
1 ATGGGCAGCAGCCATCATCATCATCATCACAGCAGCGGCCTGGTGCCGCGCGGCAGCCAT
1 M G S S H H H H H H S S G L V P R G S H

61 ATGCGCTTTGTGAACCAGCATCTGTGTGGCAGCCACCTGGTGGAAGCGCTGTATTTAGTG
21 M R F V N Q H L C G S H L V E A L Y L V

121 TGCGGCGAGCGTGGCTTCTTTTATACCCCGAAAACCAAACGTGGCATTGTGGAACAGTGT
41 C G E R G F F Y T P K T K R G I V E Q C

181 TGCACCAGTATTTGTAGCCTGTATCAGCTGGAAAATTACTGCAACTAA
61 C T S I C S L Y Q L E N Y C N * ,
 recombinant plasmid DNA fragment which encode hybrid polypeptide comprising human proinsulin sequence and which is a part of pCIM61 plasmid has the following sequence: 
1          ATGCGAAAGAAGCGGAAGAAGAAGCGTTTTGTGAACCAGCATCTGTGTGGCAGCCACCTG
1           M  R  K  K  R  K  K  K  R  F  V  N  Q  H  L  C  G  S  H  L
 
61 GTGGAAGCGCTGTATTTAGTGTGCGGCGAGCGTGGCTTCTTTTATACCCCGAAAACCAAA
21 V E A L Y L V C G E R G F F Y T P K T K

121 CGTGGCATTGTGGAACAGTGTTGCACCAGTATTTGTAGCCTGTATCAGCTGGAAAATTAC
41 R G I V E Q C C T S I C S L Y Q L E N Y

181 TGCAACTAA
61 C N * ,
 recombinant plasmid DNA fragment which encode hybrid polypeptide comprising human proinsulin sequence and which is a part of pDIM07 plasmid has the following sequence:

1          ATGGATGAAGACGAGGATGAAGCACGCTTTGTGAACCAGCATCTGTGTGGCAGCCACCTG
1           M  D  E  D  E  D  E  A  R  F  V  N  Q  H  L  C  G  S  H  L

61          GTGGAAGCGCTGTATTTAGTGTGCGGCGAGCGTGGCTTCTTTTATACCCCGAAAACCAAA
21          V  E  A  L  Y  L  V  C  G  E  R  G  F  F  Y  T  P  K  T  K

121         CGTGGCATTGTGGAACAGTGTTGCACCAGTATTTGTAGCCTGTATCAGCTGGAAAATTAC
41          R  G  I  V  E  Q  C  C  T  S  I  C  S  L  Y  Q  L  E  N  Y

181         TGCAACTAA
61          C  N  * .

2. A method according to claim 1 **characterized in that** step of *Escherichia coli* strains development is carried out using plasmid vectors, containing nucleotide sequence encoding one or several recombinant polypeptides and at the same time transcription of said nucleotide sequence encoding one or several recombinant polypeptides is under control of an inducible expression system, then culturing transformed *Escherichia coli* strain(s) and inducing the expression system to permit the expression of the recombinant polypeptide or polypeptides in the *Escherichia coli* cells and recovering of the produced recombinant polypeptide or polypeptides.

3. A method according to claim 1 **characterized in that** cell separation and disintegration and hybrid polypeptide recovery is carried out using bacterial cells or cell fragment disruption, separation of intracellular precipitate followed by washing the precipitate with nonionic detergent, while hybrid polypeptide isolation includes precipitate solubilizing in a buffer solution containing the denaturant, treatment of the hybrid polypeptide with reducing agent, refolding the hybrid polypeptide, concentrating the hybrid polypeptide by means of adsorption chromatography or ultrafiltration.

4. A method according to claim 1 **characterized in that** enzymatic cleavage of hybrid polypeptide is performed with trypsin and carboxypeptidase either simultaneously or sequentially followed by means preferably of ion exchange chromatography.

5. A method according to claim 1 **characterized in that** insulin purification after enzymatic cleavage is performed by means of ion exchange chromatography and/or reverse phase high performance liquid chromatography.
